# EUROPEAN PATENT APPLICATION

(11) **EP 4 527 307 A1**
(43) Date of publication of application: **26.03.2025**
(21) Application number: 22941930.4
(22) Date of filing: 16.05.2022
(51) Int. Cl.: A61B 8/00

(54) **ULTRASONIC PROBE AND ULTRASONIC IMAGING DEVICE**

(71) Applicant: Edan Instruments, Inc., Shenzhen, Guangdong 518000 (CN)
(72) Inventor: PENG, Minkang, Shenzhen, Guangdong 518000 (CN); OUYANG, Bo, Shenzhen, Guangdong 518000 (CN)
(74) Representative: Herrero & Asociados, S.L.
(86) International application number: PCT/CN2022/093121
(87) International publication number: WO 2023/220879

(57) **Abstract**

The present application provides an ultrasonic probe and an ultrasonic imaging device, and relates to the technical field of medical instruments. In the ultrasonic probe of the present application, a sound head assembly is accommodated in a housing; a base is accommodated in the housing; a traction rope is accommodated in the housing and is connected with the sound head assembly; a traction mechanism is accommodated in the housing, and in the traction mechanism, a traction wheel is arranged on the base and can rotate relative to the base for winding the traction rope so as to drive the sound head assembly to swing by means of the traction rope; an elastic piece is arranged on the traction wheel and is fixedly connected with the traction rope so as to adjust the tensioning force of the traction rope when the elastic piece elastically deforms. According to the present application, the elastic piece is arranged on the traction wheel, such that the ultrasonic probe can adapt to the transmission mode of the traction rope, and moreover, the elastic piece can adjust the tensioning force of the traction rope, so that the product is simple in structure and easy to assemble.

## Description

### TECHNICAL FIELD

The present disclosure relates to the field of medical apparatus technologies, and in particular to an ultrasound probe and an ultrasound imaging device.

### BACKGROUND

A common transmission method for an ultrasound probe includes a bevel gear transmission. The bevel gear transmission has high requirements for a material, processing, and assembly. In particular, an external dimension of an endoluminal ultrasound probe is small, and a diameter of a part of the endoluminal ultrasound probe protruding into a human body is usually about 25mm. An internal structure of the endoluminal ultrasound probe is complex, and it is difficult to process and assemble using the bevel gear transmission.

### SUMMARY OF THE DISCLOSURE

In a first aspect, the embodiments of the present disclosure provide an ultrasound probe. The ultrasound probe includes a housing, a sound head assembly disposed in the housing, a base disposed in the housing, a traction line disposed in the housing and connected to the sound head assembly, and a traction mechanism disposed in the housing. The traction mechanism includes a traction wheel and an elastic member. The traction wheel is disposed on the base and being rotatable relative to the base, and the traction wheel is configured to wind the traction line and drive the sound head assembly to swing through the traction line. The elastic member is disposed on the traction wheel and fixedly connected to the traction line, and the elastic member is configured to adjust tension of the traction line when the elastic member undergoes elastic deformation.

In a second aspect, the embodiments of the present disclosure provide an ultrasound imaging device including an ultrasound host and an ultrasound probe in the first aspect. The ultrasound probe is connected to the ultrasound host.

In the present disclosure, the elastic member is disposed on the traction wheel, the ultrasound probe may adapt to a transmission mode of the traction line, and tension of the traction line may be adjusted through the elastic member. Therefore, a product has a simple structure and the product is easy to assemble.

### BRIEF DESCRIPTION OF THE DRAWINGS

In order to more clearly illustrate the technical solutions in some embodiments of the present disclosure, hereinafter, the accompanying drawings that are used in the description of some embodiments will be briefly described. Obviously, the accompanying drawings in the description below are merely some embodiments of the present disclosure. For those of ordinary skill in the art, other accompanying drawings may be obtained based on these accompanying drawings without any creative efforts.
FIG. 1 is a structural schematic view of an ultrasound probe in some embodiments of the present disclosure.
FIG. 2 is a cross-sectional structural schematic view of the ultrasound probe of FIG. 1.
FIG. 3 is a schematic view illustrating a coordination relationship between the ultrasound probe and an ultrasound host in some embodiments of the present disclosure.
FIG. 4 is a structural schematic view illustrating coordination of a base, a driving mechanism, and a transmission assembly of FIG. 1.
FIG. 5 is another structural schematic view illustrating the coordination of the base, the driving mechanism, and the transmission assembly of FIG. 1.
FIG. 6 is yet another structural schematic view illustrating the coordination of the base, the driving mechanism, and the transmission assembly of FIG. 1.
FIG. 7 is a structural schematic view of a second transmission wheel of FIG. 2.
FIG. 8 is a structural schematic view of an elastic member of FIG. 6.
FIG. 9 is another structural schematic view of the elastic member of FIG. 6.
FIG. 10 is another structural schematic view of the ultrasound probe of FIG. 1.

### DETAILED DESCRIPTION

The present disclosure may be explained in detail by combining the accompanying drawings and embodiments. It should be noted that the following embodiments are only used to illustrate the present disclosure, but do not limit the scope of the present disclosure. Similarly, the following embodiments are only a part of the embodiments of the present disclosure, and not all embodiments. All other embodiments obtained by those of ordinary skill in the art without creative effort are within the scope of the present disclosure.

The reference to "embodiments" in the present disclosure means that, specific features, structures, or characteristics described in conjunction with some embodiments may be included in at least one embodiment of the present disclosure. The phrase appearing in various positions in the specification does not necessarily refer to the same embodiment, nor is it an independent or alternative embodiment that is mutually exclusive with other embodiments. Those of ordinary skill in the art explicitly and implicitly understand that the embodiments described in the present disclosure may be combined with other embodiments.

The present disclosure describes an ultrasound probe. The ultrasound probe may be configured for three-dimensional ultrasound imaging. For example, the ultrasound probe may emit and receive ultrasound waves to perform three-dimensional imaging of human tissues.

The ultrasound probe may be a 3D mechanical probe, a 4D mechanical probe, etc. That is, the ultrasound probe with a 3D imaging function or a 4D imaging function, emits an ultrasound signal to the human tissue and receives an echo signal with human tissue information.

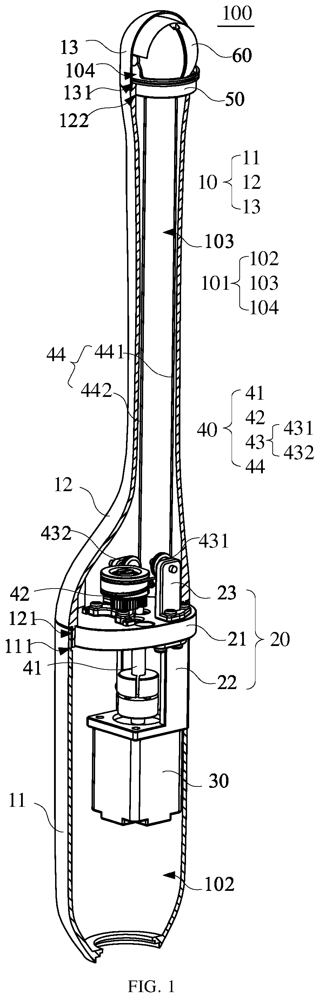

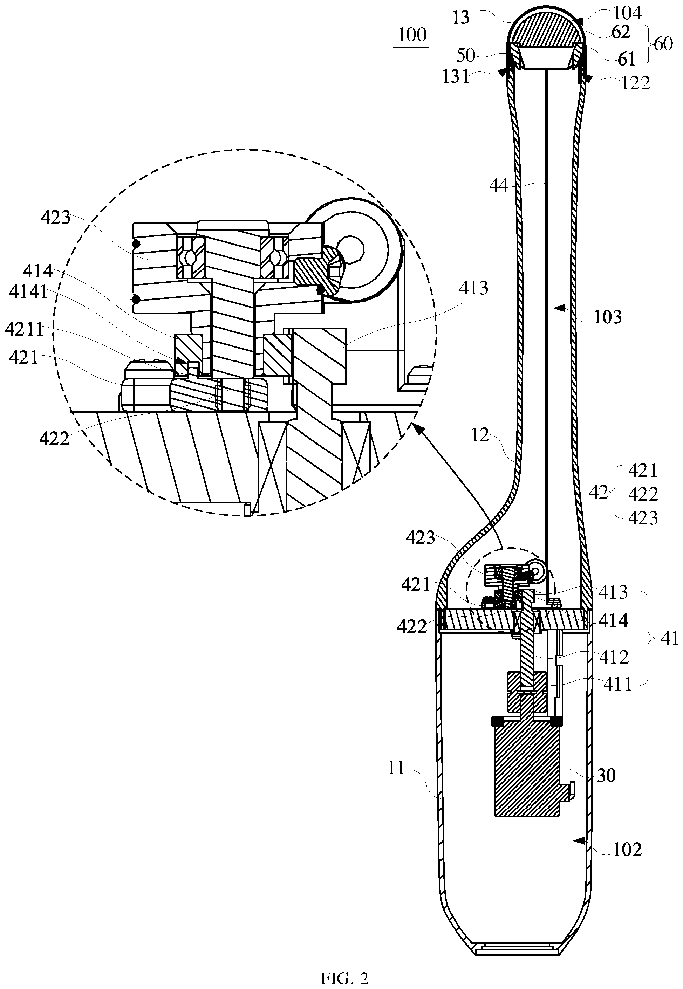

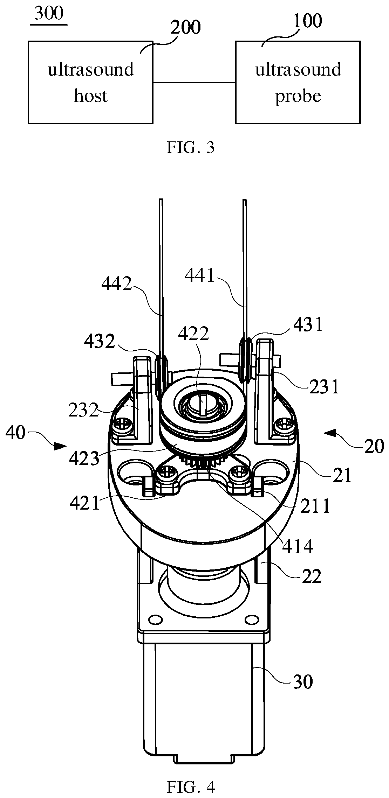

As illustrated in FIG. 1 and FIG. 2, FIG. 1 is a structural schematic view of an ultrasound probe in some embodiments of the present disclosure, and FIG. 2 is a cross-sectional structural schematic view of the ultrasound probe of FIG. 1. The ultrasound probe 100 may include a housing 10, a base 20, a driving mechanism 30, a transmission assembly 40, an installation bracket 50, and a sound head assembly 60. The housing 10 defines an installation space 101, the base 20 is disposed in the installation space 101, and the driving mechanism 30 is disposed on the base 20. The transmission assembly 40 is disposed on the base 20 and in transmission connection with the driving mechanism 30. The installation bracket 50 is disposed in the installation space 101, and the sound head assembly 60 is disposed on the installation bracket 50 and connected to the transmission assembly 40. The driving mechanism 30 serves as a power source to generate power. The sound head assembly 60 is rotatably connected to the installation bracket 50. The transmission assembly 40 transmits the power generated by the driving mechanism 30 to the sound head assembly 60, so that the driving mechanism 30 drives the sound head assembly 60 to swing back and forth. The sound head assembly 60 may perform scanning and detecting in the process of swinging back and forth. In some embodiments, as illustrated in FIG. 3, FIG. 3 is a schematic view illustrating a coordination relationship between the ultrasound probe 100 and an ultrasound host 200 in some embodiments of the present disclosure. The ultrasound probe 100, such as the driving mechanism 30 and the sound head assembly 60, may be electrically connected to the ultrasound host 200 through wired or wireless modes, so that a signal collected by the sound head assembly 60 is transmitted to the ultrasound host 200. The ultrasound host 200 may perform the three-dimensional imaging of the human tissues. In some embodiments, the ultrasound host 200 may be electrically connected to the driving mechanism 30 and the sound head assembly 60, so as to transmit a control signal to the driving mechanism 30 and the sound head assembly 60, thereby controlling normal operation of the driving mechanism 30 and the sound head assembly 60. That is, the ultrasound probe 100 and the ultrasound host 200 may form an ultrasound imaging device 300. In some embodiments, in addition to the ultrasound probe 100 and the ultrasound host 200, the ultrasound imaging device 300 may include other structures.

The ultrasound host 200 images human tissue by processing the echo signal of the ultrasound probe 100, thereby constructing a 3D image of human tissue or a 4D image of human tissue for medical analysis. For example, a 3D or 4D (3D/4D) mechanical probe may be configured for gynecological examinations. Of course, the ultrasound probe may be further configured for other purposes, such as emitting electrical stimulation to the human tissue under the signal control of the ultrasound host 200 and performing physical massage, which is not limited here.

In the description of the present disclosure, it should be noted that unless otherwise expressly specified and limited, the terms "installation", "connecting", and "connection" should be broadly understood. For example, it may be a fixed connection, a detachable connection, or an integrated connection; it may be a mechanical connection or an electrical connection; it may be a pipeline connection or a fluid line connection; it may be a direct connection or an indirect connection through an intermediate medium; it may be internal communication of two elements. For those of ordinary skill in the art, the specific meanings of the above terms may be understood according to specific situation.

As illustrated in FIG. 1, the housing 10 may include a probe handle shell 11, a probe middle shell 12, and a probe sound window 13. One end of the probe middle shell 12 is connected to an end of the probe handle shell 11, and the probe sound window 13 is disposed on the other end of the probe middle shell 12. The installation space 101 is formed by enclosing the probe handle shell 11, the probe middle shell 12, and the probe sound window 13.

The probe handle shell 11 may be made of a rigid material. An entirety of the probe handle shell 11 may have a tubular or cylindrical structure. The entirety of the probe handle shell 11 may have other structures. The probe handle shell 11 may have a shell-like structure and define an accommodating space 102. The probe handle shell 11 coordinates with the probe middle shell 12 to accommodate structures, such as the base 20, the driving mechanism 30, and the transmission assembly 40, etc. A first installation port 111 is disposed on an end of the probe handle shell 11, and the first installation port 111 is communicated with the accommodating space 102, so that the probe middle shell 12 is installed on the first installation port 111.

The probe middle shell 12 may be made of the rigid material. An entirety of the probe middle shell 12 may have the tubular or cylindrical structure. The entirety of the probe middle shell 12 may have other structures. The probe middle shell 12 may have the shell-like structure and define a receiving space 103. The probe middle shell 12 coordinates with the probe handle shell 11 to accommodate the structures, such as the base 20, the driving mechanism 30, and the transmission assembly 40, etc.

A second installation port 121 is disposed on an end of the probe middle shell 12 facing the probe handle shell 11, and the second installation port 121 is communicated with the receiving space 103. Therefore, the second installation port 121 is connected to the first installation port 111, so that the probe middle shell 12 is connected to the probe handle shell 11 and the receiving space 103 is communicated with the accommodating space 102. In some embodiments, an edge of the second installation port 121 of the probe middle shell 12 extends into the accommodating space 102 by passing through the first installation port 111, so that the probe middle shell 12 is screwed with the probe handle shell 11. Of course, a fitting mode and a connection mode between the probe middle shell 12 and the probe handle shell 11 is not limited to a screw connection. In some embodiments, the second installation port 121 is connected to the first installation port 111 by bonding, welding, clamping, or plugging, etc.

It should be pointed out that the terms "first", "second" or the like in the present disclosure are only configured to describe and cannot be understood as indicating or implying relative importance or implicit indicating the quantity of technical features indicated. Therefore, features that are defined as "first", "second" or the like may explicitly or implicitly include at least one of these features.

A third installation port 122 is disposed on the other end of the probe middle shell 12. The third installation port 122 is communicated with the receiving space 103. Therefore, the probe middle shell 12 coordinates with and is connected to the third installation port 122, so as to accommodate the structures, such as the installation bracket 50 and the sound head assembly 60, etc.

An outer surface of the end of the probe middle shell 12 facing the probe handle shell 11 smoothly transitions with an outer surface of the probe handle shell 11, so as to enhance aesthetic appeal when the probe middle shell 12 is connected to the probe handle shell 11.

In some embodiments, the probe middle shell 12 may be integrated with the probe handle shell 11.

The probe sound window 13 may be made of the rigid material. In some embodiments, the probe sound window 13 may be made of a transparent material or other materials, which is not repeated here. Of course, the material of the probe sound window 13 may be adjusted according to the needs of the ultrasound probe 100. An entirety of the probe sound window 13 may have the tubular or cylindrical structure, or other structures. The probe sound window 13 may have the shell-like structure and define a storage space 104. The probe sound window 13 may be matched with the probe middle shell 12 to accommodate the structures, such as the installation bracket 50 and the sound head assembly 60, etc.

A fourth installation port 131 is disposed on an end of the probe sound window 13. The fourth installation port 131 is communicated with the storage space 104. Therefore, the fourth installation port 131 is connect to the third installation port 122, so that the probe sound window 13 is connected to the probe middle shell 12 and the storage space 104 is communicated with the receiving space 103. In some embodiments, an edge of the fourth installation port 131 of the probe sound window 13 extends into the storage space 104 by passing through the third installation port 122, so that the probe sound window 13 is screwed with the probe middle shell 12. Of course, the fitting mode and the connection mode between the probe middle shell 12 and the probe handle shell 11 is not limited to the screw connection. In some embodiments, an edge of the third installation port 122 of the probe middle shell 12 extends into the receiving space 103 by passing through the fourth installation port 131, so that the probe middle shell 12 is screwed with the probe sound window 13. In some embodiments, the second installation port 121 is connected to the first installation port 111 by bonding, welding, clamping, or plugging, etc.

A surface of an end of the probe sound window 13 away from the fourth installation port 131 may be hemispherical, so as to enhance the aesthetic appeal. Of course, it is also to facilitate contact between the ultrasound probe 100 and the human body, or to achieve scanning and detecting of the sound head assembly 60.

In some embodiments, the probe sound window 13 may be integrated with the probe middle shell 12.

As illustrated in FIG. 1, the base 20 may include a base body 21 disposed on the probe middle shell 12, a rack 22 disposed on a side of the base body 21 facing the probe handle shell 11, and a support frame 23 installed on a side of the base body 21 facing the probe sound window 13. The base body 21 coordinates with the support frame 23 to install the transmission assembly 40. The rack 22 is configured to install the driving mechanism 30.

As illustrated in FIG. 4, FIG. 4 is a structural schematic view illustrating coordination of the base 20, the driving mechanism 30, and the transmission assembly 40 of FIG. 1. The base body 21 may be made of the rigid material. Generally, the base body 21 is in the form of a platelike structure. The base body 21 may have other structures, such as a frame structure or a columnar structure, etc. The base body 21 may be fixed in the receiving space 103 of the probe middle shell 12. In some embodiments, the base body 21 is fixed on the second installation port 121. In some embodiments, the base body 21 may be fixed on the probe middle shell 12 through screwing, welding, bonding, clamping, or plugging, etc.

A limit assembly 211 may be disposed on the side of the base body 21 facing the probe sound window 13. The limit assembly 211 coordinates with the transmission assembly 40, achieving limiting of the transmission assembly 40. In some embodiments, the limit assembly 211 may form a slide rail and be matched with the transmission assembly 40. In some embodiments, the limit assembly 211 may include a plurality of limit members, and the limit members may slidably connected to a part of the transmission assembly 40, achieving limiting of the transmission assembly 40.

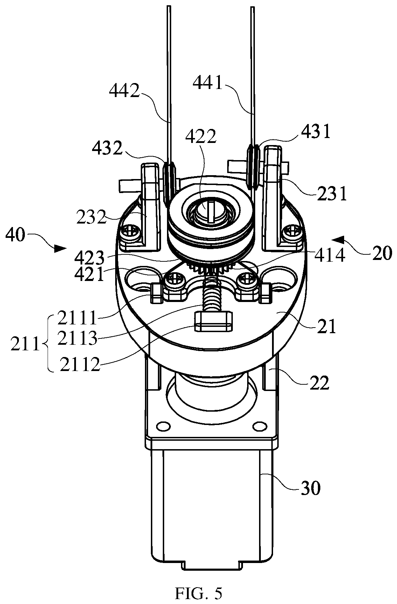

As illustrated in FIG. 5, FIG. 5 is another structural schematic view illustrating the coordination of the base 20, the driving mechanism 30, and the transmission assembly 40 of FIG. 1. The limit assembly 211 may include a limit member 2111 and a limit protrusion 2112 disposed on a surface of the base body 21, and a pre-tightening member 2113 disposed between the limit protrusion 2112 and the transmission assembly 40. In some embodiments, the limit member 2111, the limit protrusion 2112, and the pre-tightening member 2113 cooperate with each other to limit the transmission assembly 40. In some embodiments, the transmission assembly 40 may be limited only by the limit member 2111. That is, the limit protrusion 2112 may be omitted. In some embodiments, the limit member 2111 is slidably connected to the part of the transmission assembly 40, thereby achieving limiting and allowing the part of the transmission assembly 40 to slide in an extending direction of the limit member 2111. In some embodiments, both the limit member 2111 and the limit protrusion 2112 may be convex block structures. In some embodiments, when the pre-tightening member 2113 is respectively abutted against the limit protrusion 2112 and the transmission assembly 40, the part of the transmission assembly 40 is pre-limited and fixed, thereby facilitating the installation of the transmission assembly 40. In some embodiments, the pre-tightening member 2113 may be a spring, an elastic sheet, or other structures that are capable of elastic deformation. In some embodiments, the pre-tightening member 2113 may be omitted, and only the limit protrusion 2112 is abutted against the transmission assembly 40, so as to limit the transmission assembly 40.

In some embodiments, the limit member 2111 of the limit assembly 211 may be replaced by a limit sliding groove disposed on the base body 21, or other structures, which is not repeated here.

In some embodiments, the base body 21 may also be fixed in the accommodating space 102 of the probe handle shell 11 by cooperating the probe middle shell 12 with the base body 21.

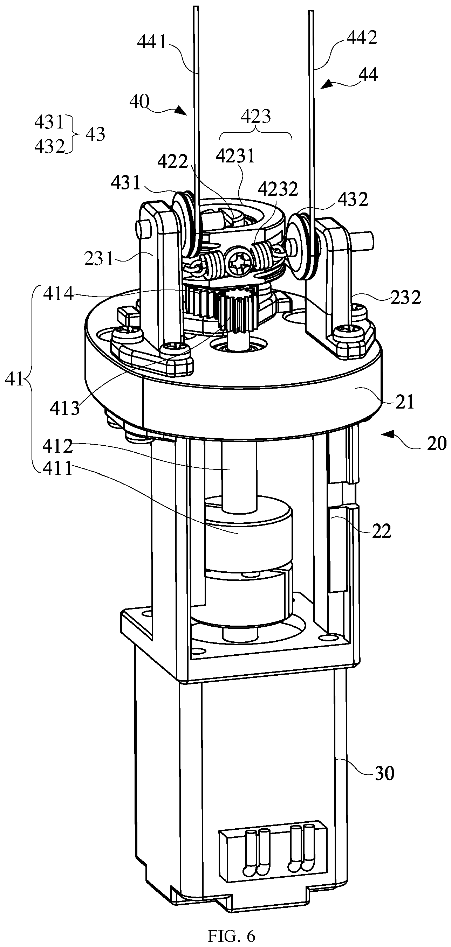

As illustrated in FIG. 1 and FIG. 6, FIG. 6 is yet another structural schematic view illustrating the coordination of the base 20, the driving mechanism 30, and the transmission assembly 40 of FIG. 1. The rack 22 is configured to install the driving mechanism 30. The rack 22 may be made of the rigid material and may have the frame structure. In some embodiments, the rack 22 may be omitted.

The support frame 23 may be made of the rigid material and may have the frame structure. The support frame 23 may have other structures. In some embodiments, the support frame 23 may include a first bracket 231 and a second bracket 232 opposite to each other. In some embodiments, at least one of the first bracket 231 and the second bracket 232 may be omitted.

As illustrated in FIG. 1 and FIG. 6, the driving mechanism 30 may be installed on the rack 22. The driving mechanism 30 may be a motor, a cylinder, or a hydraulic cylinder, etc. In some embodiments, the driving mechanism 30 may be a stepper motor. In some embodiments, the driving mechanism 30 may be directly fixed on the base body 21 instead of being disposed on the rack 22. In some embodiments, the driving mechanism 30 has an output shaft, so that the driving mechanism 30 is in transmission connection with the transmission assembly 40.

As illustrated in FIG. 1 and FIG. 6, the transmission assembly 40 may include a transmission mechanism 41, a traction mechanism 42, a steering mechanism 43, and a traction line 44. The transmission mechanism 41 is in transmission connection with the driving mechanism 30. The traction mechanism 42 is disposed on the base 20, such as the base body 21, and connected to the transmission mechanism 41. The steering mechanism 43 is disposed on the base 20, such as the support frame 23. The traction line 44 is connected to the traction mechanism 42 and the sound head assembly 60, and the traction line 44 passes by the steering mechanism 43. The transmission mechanism 41 transmits the power generated by the driving mechanism 30 to the traction mechanism 42. The traction mechanism 42 receives the power transmitted by the traction mechanism 42 to perform reciprocating motion. The steering mechanism 43 is configured to steer the traction line 44, so that the traction direction of the traction mechanism 42 is converted into the traction direction of the traction line 44. The traction line 44 is configured to pull the sound head assembly 60 and change a traction direction of the traction line 44 under the action of the steering mechanism 43. The traction line 44 is connected to the traction mechanism 42, to transmit a swinging force of the reciprocating motion of the traction mechanism 42 to the sound head assembly 60, causing the sound head assembly 60 to move.

As illustrated in FIG. 6, the transmission mechanism 41 may include a coupling 411, a transmission shaft 412, a first transmission wheel 413, and a second transmission wheel 414. The coupling 411 is disposed on the output shaft of the driving mechanism 30. The transmission shaft 412 is connected to the output shaft of the driving mechanism 30 through the coupling 411 and rotatably connected to the base 20, such as the base body 21. The first transmission wheel 413 is disposed on the transmission shaft 412 and coaxial with the transmission shaft 412. The second transmission wheel 414 is disposed on the traction mechanism 42 and in transmission connection with the first transmission wheel 413. The transmission shaft 412 may penetrate the base 20, such as the base body 21, and be rotatably connected to the base 20, such as the base body 21. In some embodiments, the transmission shaft 412 may be rotatably connected to the base 20, such as the base body 21, through a bearing. In some embodiments, a sealing element may be disposed between the transmission shaft 412 and the base 20, such as the base body 21, achieving a sealing effect. In some embodiments, the transmission shaft 412 may not be rotatably connected to the base 20, such as the base body 21. In some embodiments, the transmission shaft 412 may be integrated with the output shaft of the driving mechanism 30, so that the coupling 411 may be omitted in some scenarios. In some embodiments, the transmission shaft 412 may also be in transmission connection with the output shaft of the driving mechanism 30 through other structures, such as a gear or a drive belt, etc.

The first transmission wheel 413 is located on the side of the base 20, such as the base body 21, facing the probe sound window 13. The first transmission wheel 413 may transmit the power generated by the driving mechanism 30 to the second transmission wheel 414, and then the power generated by the driving mechanism 30 is transmitted to the traction mechanism 42. In some embodiments, the first transmission wheel 413 may be a rotating wheel or a gear, so that the first transmission wheel 413 is connected to the second transmission wheel 414 through a transmission belt, gear meshing, etc.

In some embodiments, the first transmission wheel 413 may be omitted, and the transmission shaft 412 is in transmission connection with the second transmission wheel 414 through other structures. In some embodiments, the transmission shaft 412 may be directly connected to the second transmission wheel 414.

In some embodiments, the first transmission wheel 413 is a synchronous wheel.

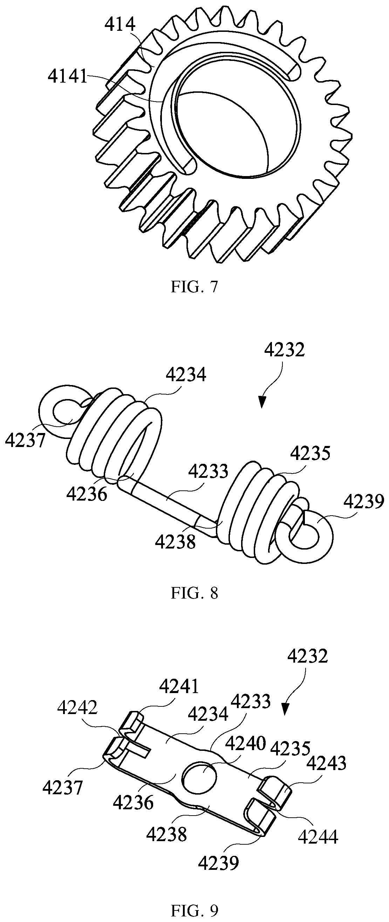

As illustrated in FIG. 2 and FIG. 7, FIG. 7 is a structural schematic view of the second transmission wheel 414 of FIG. 2. The second transmission wheel 414 is installed on the traction mechanism 42 and in transmission connection with the first transmission wheel 413. The second transmission wheel 414 is located on the side of the base 20, such as the base body 21, facing the probe sound window 13. The second transmission wheel 414 may be connected to the first transmission wheel 413 through the gear meshing or the transmission belt, etc., thereby achieving power transmission between the second transmission wheel 414 and the first transmission wheel 413.

The second transmission wheel 414 may be the rotating wheel, the gear, or the synchronous wheel.

A side of the second transmission wheel 414 facing the base 20, such as the base body 21, defines a limit groove 4141. The limit groove 4141 cooperates with the traction mechanism 42, to achieve the limitation of a rotation angle of the second transmission wheel 414. In some embodiments, a setting position of the limit groove 4141 may be changed, as long as the limit groove 4141 may cooperate with the traction mechanism 42 to limit the rotation angle of the second transmission wheel 414. In some embodiments, the limit groove 4141 may be disposed on the traction mechanism 42. In some embodiments, the limit groove 4141 is arc-shaped and distributed on the second transmission wheel 414.

As illustrated in FIG. 2, FIG. 4, and FIG. 5. The traction mechanism 42 may include an adjustment bracket 421, a traction shaft 422, and a traction wheel 423. The adjustment bracket 421 is disposed on the base 20, such as the base body 21. The traction shaft 422 is disposed on the adjustment bracket 421 and interference fit with the transmission mechanism 41, such as the second transmission wheel 414. The traction wheel 423 is fixedly connected to the traction shaft 422. The traction wheel 423 is coaxially disposed with the transmission mechanism 41, such as the second transmission wheel 414. The traction wheel 423 is connected to the traction line 44 to transmit the power to the traction line 44.

The adjustment bracket 421 may be made of the rigid material. The adjustment bracket 421 may be fixed to the base 20, such as the base body 21, by screwing, clamping, welding, or bonding, etc. That is, the adjustment bracket 421 has a locked state that the adjustment bracket 421 is fixed relative to the base 20, such as the base body 21. The adjustment bracket 421 has an unlocked state that the adjustment bracket 421 is slideable relative to the base 20, such as the base body 21.

The adjustment bracket 421 is located on the side of the base 20, such as the base body 21, facing the probe sound window 13. In some embodiments, the adjustment bracket 421 is disposed between the transmission mechanism 41, such as the second transmission wheel 414, and the base 20, such as the base body 21.

In some embodiments, the adjustment bracket 421 may be limited by the limit assembly 211. In some embodiments, the adjustment bracket 421 may be clamped between two limit members 2111. Therefore, the adjustment bracket 421 may move close to or away from the transmission mechanism 41, such as the first transmission wheel 413, in an extending direction of the limit member 2111. Therefore, the fit clearance between the first transmission wheel 413 and the second transmission wheel 414 may be adjusted. That is, the two limit members 2111 may form a sliding rail and be in slip connection with the adjustment bracket 421. In some embodiments, the limit sliding groove may be defined on the side of the adjustment bracket 421 facing the base 20, such as the base body 21, so as to accommodate the limit member 2111. Therefore, the limit member 2111 moves close to or away from the transmission mechanism 41, such as the first transmission wheel 413, in an extending direction of the limit chute, so as to adjust the fit clearance between the first transmission wheel 413 and the second transmission wheel 414. In some embodiments, in the unlocked state where the adjustment bracket 421 is slideable relative to the base 20, such as the base body 21, the fit clearance between the first transmission wheel 413 and the second transmission wheel 414 may be adjusted. In this case, a distance between an axis of the first transmission wheel 413 and an axis of the second transmission wheel 414 may be adjusted, thereby preventing the mechanical transmission from making noises during the use of the ultrasound probe 100.

In some embodiments, the side of the adjustment bracket 421 away from the transmission mechanism 41, such as the first transmission wheel 413, may be butted with an end of the pre-tightening member 2113. Therefore, in the unlocked state where the adjustment bracket 421 is slideable relative to the base 20, such as the base body 21, the adjustment bracket 421 coordinates with the pre-tightening member 2113, so that the first transmission wheel 413 is tightly fit with the second transmission wheel 414, further fixing the adjustment bracket 421 relative to the base 20, such as the base body 21, thereby allowing the adjustment bracket 421 to be in the locked state.

In some embodiments, the adjusting bracket 421 may be directly disposed on the limit assembly 211, and may be fixed in the locked state by screwing, clamping, or other limit modes.

A limit sliding member 4211 is disposed on the side of the adjustment bracket 421 facing the probe sound window 13. The limit sliding member 4211 cooperates with the transmission mechanism 41, such as the second transmission wheel 414, so as to limit and adjust the rotation angle (also known as a deflection angle) of the second transmission wheel 414. In some embodiments, the limit sliding member 4211 extends into the limit groove 4141 and slides in the extending direction of the limit groove 4141, thereby adjusting the rotation angle of the second transmission wheel 414. In some embodiments, the limit sliding member 4211 may be the sliding block. It may be understood that when adjusting the bracket 421 cooperates with the transmission mechanism 41, such as the second transmission wheel 414, for limiting, it is not limited to the cooperation relationship between the limiting sliding piece 4211 and the limiting groove 4141, it may have other cooperation relationships. In addition, positions of the limit sliding member 4211 and the limit groove 4141 may also be interchanged.

In some embodiments, the adjustment bracket 421 may not be a part of the traction mechanism 42.

The traction shaft 422 is located on the side of the base 20, such as the base body 21, facing the probe sound window 13. The traction shaft 422 may be connected to the adjustment bracket 421 by a fixed connection or a rotating connection, etc.

The traction shaft 422 may pass through the second transmission wheel 414, and the traction shaft 422 may be coaxially disposed with the second transmission wheel 414. In some embodiments, the traction shaft 422 may be fixedly connected to the second transmission wheel 414 through interference fit. In some embodiments, the traction shaft 422 may be directly rotatably connected to the second transmission wheel 414.

In some embodiments, the traction shaft 422 may be directly fixed on the adjustment bracket 421. In some embodiments, the traction shaft 422 may also be rotatably connected to the adjustment bracket 421. In some embodiments, the traction shaft 422 may be rotatably connected to the adjustment bracket 421 through the bearing.

An axis of the traction shaft 422 is parallel to an axis of the transmission mechanism 41, such as the transmission shaft 412.

The traction wheel 423 is coaxially disposed with the traction shaft 422 and rotatably connected to the traction shaft 422. In some embodiments, the traction wheel 423 may be connected to the traction shaft 422 through the bearing.

The traction wheel 423 may be coaxially disposed with the second transmission wheel 414, so that the second transmission wheel 414 is sleeved on the traction wheel 423. Therefore, the second transmission wheel 414 is indirectly rotatably connected to the traction shaft 422. In some embodiments, the traction wheel 423 may be fixedly connected to the second transmission wheel 414, so that the traction wheel 423 rotates relative to the traction shaft 422 together with the second transmission wheel 414. In some embodiments, the traction wheel 423 may be fixedly connected to the second transmission wheel 414 and the traction shaft 422, so that the traction wheel 423, the second transmission wheel 414, and the traction shaft 422 rotate together relative to the adjustment bracket 421.

In some embodiments, at least two of the second transmission wheel 414, the traction shaft 422, and the traction wheel 423 may be integrated. That is, the traction shaft 422 may be a part of the traction wheel 423.

It may be understood that the limit sliding member 4211 and the limit groove 4141 are not limited to being disposed on the adjustment bracket 421 and the second transmission wheel 414. The limit sliding member 4211 and the limit groove 4141 may also be disposed on any two of the base body 21, the adjustment bracket 421, the second transmission wheel 414, the traction shaft 422, and the traction wheel 423 that rotate relative to each other. In some embodiments, the limit sliding member 4211 is disposed on the adjustment bracket 421, and the second transmission wheel 414 is disposed on the traction shaft 422. The second transmission wheel 414, the traction shaft 422, and the traction wheel 423 are fixed to each other. The traction shaft 422 is rotatably connected to the adjustment bracket 421. In some embodiments, the limit sliding member 4211 is disposed on the traction shaft 422, and the second transmission wheel 414 is disposed on the traction wheel 423.

As illustrated in FIG. 6, the traction wheel 423 may include a traction wheel body 4231 and an elastic member 4232. The traction wheel body 4231 is rotatably connected to the traction shaft 422 and fixedly connected to the second transmission wheel 414. The elastic member 4232 is disposed on the traction wheel body 4231 and configured for being fixedly connected to the traction line 44.

A helical groove may be disposed on the traction wheel body 4231 to wind the traction line 44.

The elastic member 4232 is configured to ensure the tension of the traction line 44 and further serves as a buffer, so as to prevent the traction line 44 from breaking. In some embodiments, a middle part of the elastic member 4232 is fixed on the traction wheel body 4231, and two ends of the elastic member 4232 are connected to the traction line 44. In some embodiments, the elastic member 4232 may be the spring, the elastic sheet, or other structures that are capable of elastic deformation.

As illustrated in FIG. 8 and FIG. 9, FIG. 8 is a structural schematic view of an elastic member 4232 of FIG. 6, and FIG. 9 is another structural schematic view of the elastic member 4232 of FIG. 6. The elastic member 4232 may include a fixed part 4233, a first connection part 4234, and a second connection part 4235. The first connection part 4234 and the second connection part 4235 are disposed on two opposite sides of the fixed part 4233, respectively. The first connection part 4234 and the second connection part 4235 are respectively connected to the fixed part 4233, so that the first connection part 4234 and the second connection part 4235 may be integrated in some scenarios.

The fixed part 4233 may be fixed on the traction wheel body 4231. In some embodiments, the fixed part 4233 may be fixed on the traction wheel body 4231 by bonding, welding, or screwing, etc. The first connection part 4234 includes a first end 4236 close to the fixed part 4233 and a second end 4237 away from the fixed part 4233. The first end 4236 is connected to the fixed part 4233, and the second end 4237 is connected to the traction line 44. The second connection part 4235 is disposed on a side of the fixed part 4233 away from the first connection part 4234. The second connection part 4235 includes a third end 4238 close to the fixed part 4233 and a fourth end 4239 away from the fixed part 4233. The third end 4238 is connected to the fixed part 4233, and the fourth end 4239 is connected to the traction line 44.

In some embodiments, a central axis of the first connection part 4234 is collinear with a central axis of the second connection part 4235. It may be understood that the central axis may be a symmetry axis (such as a symmetry axis of center symmetry, or a symmetry axis of axis symmetry), or an axis on which a center of the connection part is located.

As illustrated in FIG. 8, each of the first connection part 4234 and the second connection part 4235 may be an elastic element, such as the spring.

As illustrated in FIG. 9, each of the first connection part 4234 and the second connection part 4235 may be the elastic element, such as the spring sheet.

As illustrated in FIG. 9, the fixed part 4233 defines a positioning hole 4240, so that the fixed part 4233 is fixed on the traction wheel body 4231 through the positioning hole 4240 in conjunction with the screws, the bolt, etc.

As illustrated in FIG. 9, the second end 4237 of the first connection part 4234 is further connected to a first hook part 4241, so that the first connection part 4234 is connected to the traction line 44. In some embodiments, the first hook part 4241 is bent and extends from the second end 4237 towards the second connection part 4235, which facilitates the connection of the first connection part 4234 with the traction line 44.

In some embodiments, the second end 4237 of the first connection part 4234 defines a first fastening groove 4242, so as to stuck and fix the traction line 44. In some embodiments, the first fastening groove 4242 extends from the second end 4237 along an extending direction of the first hook part 4241, and penetrates the first hook part 4241.

The fourth end 4239 of the second connection part 4235 is further connected to a second hook part 4243, so that the second connection part 4235 is connected to the traction line 44. In some embodiments, the second hook part 4243 is bent and extends from the fourth end 4239 towards the first connection part 4234, which facilitates the connection of the second connection part 4235 with the traction line 44.

In some embodiments, the fourth end 4239 of the second connection part 4235 defines a second fastening groove 4244, so as to stuck and fix the traction line 44. In some embodiments, the second fastening groove 4244 extends from the fourth end 4239 along an extending direction of the second hook part 4243, and penetrates the second hook part 4243.

In some embodiments, the elastic member 4232 may not be a part of the traction wheel 423.

As illustrated in FIG. 6, the steering mechanism 43 is installed on the support frame 23, such as the first bracket 231 and the second bracket 232, so as to wind the traction line 44, thereby achieving a change in a direction of the traction force. The steering mechanism 43 may include a first steering wheel 431 and a second steering wheel 432. The first steering wheel 431 is rotatably connected to the support frame 23, such as the first bracket 231. The second steering wheel 432 is rotatably connected to the support frame 23, such as the second bracket 232.

In some embodiments, an angle may be formed between an axis of rotation of the first steering wheel 431 and an axis of rotation of the traction wheel 423. In some embodiments, the angle between the axis of rotation of the first steering wheel 431 and the axis of rotation of the traction wheel 423 may range from 0 degrees to 90 degrees. In some embodiments, an angle may be formed between an axis of rotation of the second steering wheel 432 and the axis of rotation of the traction wheel 423. In some embodiments, the angle between the axis of rotation of the second steering wheel 432 and the axis of rotation of the traction wheel 423 may range from 0 degrees to 90 degrees.

In some embodiments, the steering mechanism 43 may be omitted.

As illustrated in FIG. 1 and FIG. 6, the traction line 44 may be a rope, a chain, or a transmission belt, etc. The traction line 44 may be wound around the sound head assembly 60. The traction line 44 may also be wound around the transmission assembly 40, such as the traction wheel 423. In some embodiments, the traction line 44 may be accommodated in the helical groove on the traction wheel 423. One end of the traction line 44 is connected to one end of the transmission assembly 40, such as the elastic member 4232, and the other end of the traction line 44 is connected to the other end of the transmission assembly 40, such as the elastic member 4232. In some embodiments, when the elastic member 4232 is omitted, the traction line 44 may have a circular structure.

In some embodiments, the traction line 44 may include a first rope 441 and a second rope 442. One end of the first rope 441 is connected to the sound head assembly 60, and the other end of the first rope 441 is connected to one end of the elastic member 4232. The first rope 441 is wound around the steering mechanism 43, such as the first steering wheel 431. One end of the second rope 442 is connected to the sound head assembly 60, and the other end of the second rope 442 is connected to the other end of the elastic member 4232. The second rope 442 is wound around the steering mechanism 43, such as the second steering wheel 432.

In some embodiments, the first rope 441 may be connected to the second end 4237 of the first connection part 4234 of the elastic member 4232 in FIG. 7 and FIG. 8.

In some embodiments, the second rope 442 may be connected to the fourth end 4239 of the second connection part 4235 of the elastic member 4232 in FIG. 7 and FIG. 8.

As illustrated in FIG. 1 and FIG. 2, the installation bracket 50 may be made of the rigid material. In some embodiments, the material of the installation bracket 50 may be the same as the material of each of the probe handle shell 11, the probe middle shell 12, and the probe sound window 13. The installation bracket 50 may be fixed in the receiving space 103 of the probe middle shell 12. In some embodiments, the installation bracket 50 is fixed on the third installation port 122. In some embodiments, the installation bracket 50 may be fixed to the probe middle shell 12 through screwing, welding, bonding, clamping, or plugging, etc.

In some embodiments, the installation bracket 50 may also be fixed in the storage space 104 of the probe sound window 13 by cooperating the probe middle shell 12 with the installation bracket 50.

In some embodiments, the installation bracket 50 may be a part of the housing 10. The installation bracket 50 may also be integrated with the housing 10.

In some embodiments, the containment space 104 may be formed by enclosing the installation bracket 50 and the probe sound window 13.

As illustrated in FIG. 2, the sound head assembly 60 may include a rotating bracket 61 and a transducer 62. The rotating bracket 61 is disposed on the installation bracket 50. The transducer 62 is disposed on a side of the rotating bracket 61 away from the installation bracket 50. The rotating bracket 61 may be rotatably connected to the installation bracket 50 through a rotating shaft, so that the rotating bracket 61 may rotate relative to the installation bracket 50. The transducer 62 may rotate together with the rotating bracket 61 relative to the installation bracket 50. The traction line 44 may be wound around the rotating bracket 61. In some embodiments, the traction line 44 may be sleeved on the rotating bracket 61. In some embodiments, the rotating bracket 61 may be connected to an end of the first rope 441 and the second end 4237, respectively.

As illustrated in FIG. 1, FIG. 2, FIG. 4, FIG. 5, and FIG. 6, the driving mechanism 30 may drive the first transmission wheel 413 to rotate, thereby synchronously rotating the second transmission wheel 414 and the traction wheel 423, further winding one end of the traction line 44 and releasing the other end of the traction line 44. Therefore, the traction line 44 drives the sound head assembly 60 to rotate relative to the installation bracket 50, thereby achieving the reciprocating swinging motion of the sound head assembly 60.

The steering mechanism 43, such as the first steering wheel 431, the second steering wheel 432, achieves the steering of the traction line 44.

The cooperation between the limit sliding member 4211 and the limit groove 4141 limits the rotation angle of the traction wheel 423, thereby achieving the adjustment of the swing angle of the sound head assembly 60.

The adjustment bracket 421 slides close to or away from the first transmission wheel 413 in the extending direction of the limit member 2111, which may adjust the fit clearance between the first transmission wheel 413 and the second transmission wheel 414, and adjust the rotation angle of the traction wheel 423 and also adjust the tension of the traction line 44. It may be understood that the elastic member 4232 may avoid excessive tension of the traction line 44, and the excessive tension of the traction line 44 is caused by the sliding of the adjustment bracket 421 on the limit member 2111.

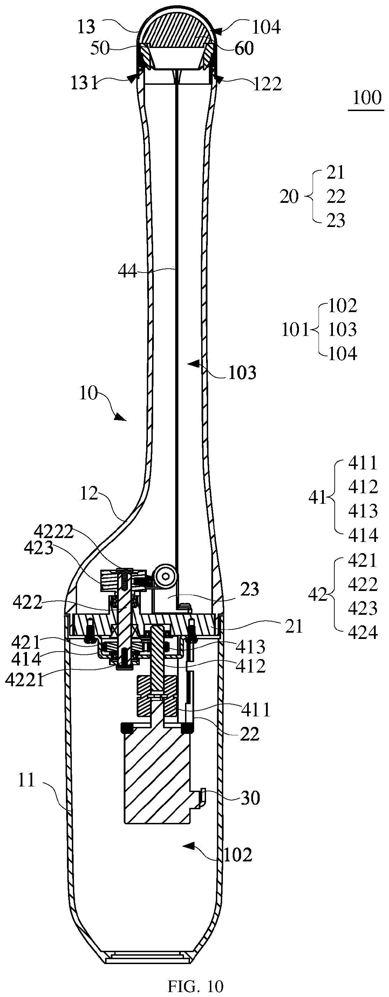

As illustrated in FIG.10, FIG. 10 is another structural schematic view of the ultrasound probe 100 of FIG. 1. The ultrasound probe 100 may include the housing 10, the base 20, the driving mechanism 30, the transmission assembly 40, the installation bracket 50, and the sound head assembly 60. The housing 10 defines the installation space 101, the base 20 is disposed in the installation space 101, and the driving mechanism 30 is disposed on the base 20. The transmission assembly 40 is disposed on the base 20 and in transmission connection with the driving mechanism 30. The installation bracket 50 is disposed in the installation space 101, and the sound head assembly 60 is disposed on the installation bracket 50 and connected to the transmission assembly 40. The driving mechanism 30 serves as the power source to generate power. The sound head assembly 60 is rotatably connected to the installation bracket 50. The transmission assembly 40 transmits the power generated by the driving mechanism 30 to the sound head assembly 60, so that the driving mechanism 30 drives the sound head assembly 60 to swing back and forth. The sound head assembly 60 may perform scanning and detecting in the process of swinging back and forth. In some embodiments, the sound head assembly 60 may be electrically connected to the ultrasound host 200, so that the signal collected by the sound head assembly 60 is transmitted to the ultrasound host 200. The ultrasound host 200 may perform the three-dimensional imaging of the human tissues. In some embodiments, the ultrasound host 200 may be electrically connected to the driving mechanism 30 and the sound head assembly 60, so as to transmit the control signal to the driving mechanism 30 and the sound head assembly 60, thereby controlling normal operation of the driving mechanism 30 and the sound head assembly 60.

The coordination relationship between the housing 10, the base 20, the driving mechanism 30, the installation bracket 50, and the sound head assembly 60, and the setting method of each structure, may refer to the descriptions in the above embodiments, which is not repeated here.

The partial structure of the transmission assembly 40 may refer to the descriptions in the above embodiments, which is not repeated here. That is, the cooperation relationship between the transmission mechanism 41, the traction mechanism 42, the steering mechanism 43, and the traction line 44 may refer to the descriptions in the above embodiments, which is not repeated here. Only differences of some structures may be explained here.

As illustrated in FIG. 10, the transmission shaft 412 may be rotatably connected to the base 20, such as the base body 21. The transmission shaft 412 is located on the side of the base body 21 away from the probe sound window 13. In some embodiments, the transmission shaft 412 may be rotatably connected to the base 20, such as the base body 21, through the bearing. In some embodiments, the first transmission wheel 413 and the second transmission wheel 414 are located on the side of the base 20, such as the base body 21, away from the probe sound window 13.

In some embodiments, the traction mechanism 42 may include the adjustment bracket 421, the traction shaft 422, and the traction wheel 423. The adjustment bracket 421 is disposed on the side of the base 20, such as the base body 21, away from the probe sound window 13. The traction shaft 422 penetrates the base 20, such as the base body 21. The traction shaft 422 is rotatably connected to the base 20, such as the base body 21. The traction wheel 423 is disposed on the side of the base 20, such as the base body 21, facing the probe sound window 13.

In some embodiments, the first transmission wheel 413 and the second transmission wheel 414 are synchronous wheels, and synchronous transmission of the first transmission wheel 413 and the second transmission wheel 414 is achieved through the transmission belt.

In some embodiments, a fastener 4221 is disposed on one end of the traction shaft 422, and a fastener 4222 is disposed on the other end of the traction shaft 422.

The fastener 4221 is fixed on the traction shaft 422 and located on an end of the second transmission wheel 414 away from the traction wheel 423, so that the fastener 4221 may limit the traction shaft 422 and prevent the traction shaft 422 from moving in an axial direction of the traction shaft 422. In some embodiments, the rotational connection and/or limit between the traction shaft 422 and the adjustment bracket 421 may be achieved through the fastener 4221.

The fastener 4222 is fixed on the traction shaft 422 and located on an end of the traction wheel 423 away from the second transmission wheel 414, so that the fastener 4221 may limit the traction shaft 422 and prevent the traction shaft 422 from moving in the axial direction of the traction shaft 422. In some embodiments, the fixed connection and/or limit between the traction shaft 422 and the traction wheel 423 may be achieved through the fastener 4221.

It may be understood that setting modes of the fastener 4221 and the fastener 4222 may be applied to above embodiments, so as to limit movement of the traction shaft 422 along the axial direction of the traction shaft 422.

In the embodiments provided by the present disclosure, it should be understood that, the disclosed methods and devices may be implemented in other manners. For example, the device embodiments described above are only illustrative. For example, the division of the modules or units is only a logical function division. In actual implementation, other division manners may be possible. For example, a plurality of units or components may be combined or integrated into another system, or some features may be omitted or may not be implemented.

The units described as separate components may or may not be physically separated, and the components displayed as units may or may not be physical units. That is, they may be located in one place, or may also be distributed on a plurality of network units. Some or all of the units may be selected according to actual requirements to implement the solutions of the embodiments.

In addition, the respective functional units in various embodiments of the present disclosure may be integrated into one processing unit, or each unit may exist alone physically, or two or more units may be integrated into one unit. The above-mentioned integrated unit may be realized in the form of hardware or in the form of a software functional unit.

The above descriptions are only some embodiments of the present disclosure, and are not intended to limit the scope of the present disclosure. Any equivalent structure or equivalent flow transformation made by using the contents of the specification and accompanying drawings of the present disclosure, or directly or indirectly applied to other related technical fields, is included in the scope of the patent protection of the present disclosure.

## Claims

1. An ultrasound probe, **characterized by** comprising:
a housing;
a sound head assembly, disposed in the housing;
a base, disposed in the housing;
a traction line, disposed in the housing and connected to the sound head assembly; and
a traction mechanism, disposed in the housing and comprising:
a traction wheel, disposed on the base and being rotatable relative to the base, wherein the traction wheel is configured to wind the traction line and drive the sound head assembly to swing through the traction line; and
an elastic member, disposed on the traction wheel and fixedly connected to the traction line, wherein the elastic member is configured to adjust tension of the traction line when the elastic member elastically deforms.

2. The ultrasound probe according to claim 1, wherein the elastic member comprises:
a fixed part, fixedly connected to the traction wheel;
a first connection part, comprising a first end close to the fixed part and a second end away from the fixed part, wherein the first end is connected to the fixed part and the second end is connected to the traction line; and
a second connection part, disposed on a side of the fixed part away from the first connection part, wherein the second connection part comprises a third end close to the fixed part and a fourth end away from the fixed part, the third end is connected to the fixed part, and the fourth end is connected to the traction line.

3. The ultrasound probe according to claim 2, wherein the fixed part, the first connection part, and the second connection part are integrated.

4. The ultrasound probe according to claim 3, wherein a central axis of the first connection part is collinear with a central axis of the second connection part.

5. The ultrasound probe according to claim 3, wherein the fixed part defines a positioning hole, and the elastic member is fixed on the traction wheel through the positioning hole; both the first connection part and the second connection part are elastic elements; and the positioning hole comprises two opposite sides, the first connection part is disposed on one side of the positioning hole, and the second connection part is disposed on the other side of the positioning hole.

6. The ultrasound probe according to claim 4 or 5, wherein the second end of the first connection part is further connected to a first hook part, and the traction line is connected to the first hook part; and
The fourth end of the second connection part is further connected to a second hook, and the traction line is connected to the second hook.

7. The ultrasound probe according to claim 6, wherein the first hook part is bent and extends from the second end towards the second connection part, and the second hook part is bent and extends from the fourth end towards the first connection part.

8. The ultrasound probe according to claim 7, wherein the elastic member further defines a first fastening groove and a second fastening groove; and one end of the traction line is clamped in the first fastening groove, and the other end of the traction line is clamped in the second fastening groove; and
the first fastening groove extends from the second end along an extending direction of the first hook part and penetrates the first hook part, and the second fastening groove extends from the fourth end along an extending direction of the second hook part and penetrates the second hook part.

9. The ultrasound probe according to any one of claims 1-5, further comprising:
a transmission mechanism, disposed in the housing and being rotatable relative to the housing, wherein the transmission mechanism is fixedly connected to the traction mechanism, and configured for driving the traction mechanism to rotate relative to the base.

10. The ultrasound probe according to claim 9, further comprising:
a driving mechanism, disposed in the housing and connected to the transmission mechanism, wherein the driving mechanism comprises an output shaft; and the transmission mechanism comprises:
a transmission shaft, connected to the output shaft of the driving mechanism, wherein the transmission shaft is rotatable relative to the base under driving of the driving mechanism;
a first transmission wheel, sleeved on the transmission shaft and rotating with rotation of the transmission shaft; and
a second transmission wheel, wherein the second transmission wheel is in transmission connection with the first transmission wheel; and the second transmission wheel is interference fit with the traction mechanism, and configured to drive the traction wheel to rotate.

11. The ultrasound probe according to claim 10, wherein each of the first transmission wheel and the second transmission wheel is a gear, and the second transmission wheel is in interference fit with the traction wheel and meshes with the first transmission wheel for transmission.

12. The ultrasound probe according to claim 11, further comprising:
an adjusting bracket, disposed between the second transmission wheel and the base, wherein the adjusting bracket comprises a locked state that the adjustment bracket is fixed relative to the base and an unlocked state that the adjustment bracket is slideable relative to the base; and the adjusting bracket is rotatably connected to the second transmission wheel, and configured for driving the second transmission wheel to slide with the adjusting bracket relative to the base when the adjusting bracket is in the unlocked state.

13. The ultrasound probe according to claim 12, wherein one of the second transmission wheel and the adjustment bracket defines a limit groove, and the other of the second transmission wheel and the adjustment bracket is provided with a limit sliding member; and the limit sliding member is configured to cooperates with the limit groove, so that a rotation angle of the traction wheel relative to the adjustment bracket is adjusted.

14. The ultrasound probe according to claim 12 or 13, wherein a limit protrusion is disposed on a surface of the base close to the adjustment bracket, and configured for limiting the adjustment bracket.

15. The ultrasound probe according to claim 14, wherein a pre-tightening member is further disposed between the limit protrusion and the adjustment bracket, so that the first transmission wheel is meshed with the second transmission wheel.

16. The ultrasound probe according to claim 12 or 13, wherein one of the base and the adjustment bracket defines a limit sliding groove, and the other of the base and the adjustment bracket is provided with a limit member; and the limit member is configured to slidably connected to the limit sliding groove, so that the adjustment bracket is slidable relative to the base when the adjusting bracket is in the unlocked state.

17. The ultrasound probe according to claim 12 or 13, wherein the traction wheel comprises:
a traction wheel body, disposed on the base and being rotatable relative to the adjustment bracket, wherein the traction wheel body is configured for winding the traction line; and
a traction shaft, extending and passing through the traction wheel body, wherein the traction shaft is inserted into the adjustment bracket.

18. The ultrasound probe according to claim 10, wherein the first transmission wheel and the second transmission wheel are synchronous wheels; the transmission mechanism further comprises a transmission belt, and the transmission belt is connected to the first transmission wheel and the second transmission wheel; and the transmission belt is configured to drive the second transmission wheel to rotate synchronously with the first transmission wheel;
the traction wheel comprises:
a traction wheel body, disposed on the base and being rotatable relative to the base, wherein the traction wheel body is configured for winding the traction line; and
a traction shaft, extending and passing through the traction wheel body, wherein the traction shaft is inserted and fixed into the second transmission wheel, and the traction shaft is configured to rotate with rotation of the second transmission wheel and further drive the traction wheel body to rotate.

19. The ultrasound probe according to claim 18, wherein each of two opposite sides of the traction shaft along an axial direction of the traction shaft is provided with a fastener, and the fastener is configured to limit movement of the traction shaft along the axial direction of the traction shaft.

20. The ultrasound probe according to any one of claims 1-5, further comprising:
a steering mechanism, disposed on the base, wherein the steering mechanism is configured to steer the traction line after being wound out by the traction wheel, so that the traction line is connected to the sound head assembly.

21. The ultrasound probe according to claim 20, wherein the steering mechanism comprises a first steering wheel and a second steering wheel spaced apart from each other and disposed on the base; and
the traction line comprises:
a first rope, wherein one end of the first rope is connected to the sound head assembly, and the other end of the first rope is wound around the traction wheel after passing by the first steering wheel; and the first rope is connected to one end of the elastic member; and
a second rope, wherein one end of the second rope is connected to the sound head assembly, and the other end of the second rope is wound around the traction wheel after passing by the second steering wheel; and second rope is connected to the other end of the elastic member.

22. The ultrasound probe according to any one of claims 1-5, wherein the housing comprises:
a probe handle shell;
a probe middle shell, wherein one end of the probe middle shell is connected to an end of the probe handle shell; and
a probe sound window, disposed on the other end of the probe middle shell, wherein the sound head assembly is accommodated in the probe sound window;
wherein the probe handle shell, the probe middle shell, and the probe sound window are enclosed to form an installation space, so as to accommodate the base, the traction wheel, and the traction line.

23. The ultrasound probe according to claim 22, wherein the housing further comprises:
an installation bracket, accommodated in the probe sound window, wherein the installation bracket and the probe sound window are enclosed to form a storage space;
the sound head assembly comprises:
a rotating bracket, disposed on the installation bracket and accommodated in the storage space, wherein the rotating bracket is rotatable relative to the installation bracket; and
a transducer, disposed on a side of the rotating bracket away from the installation bracket and configured to rotate with rotation of the rotating bracket.

24. An ultrasound imaging device, **characterized by** comprising:
an ultrasound host; and
an ultrasound probe of any one of claims 1 to 23, wherein the ultrasound probe is connected to the ultrasound host.
